# EUROPÄISCHE PATENTANMELDUNG

(11) **EP 0 857 781 A2**
(43) Veröffentlichungstag der Anmeldung: **12.08.1998**
(21) Anmeldenummer: 98102014.2
(22) Anmeldetag: 05.02.1998
(51) Int. Cl.: C12N 15/11, C12N 15/85

(54) **Promotor des humanen Endoglingens sowie seine Verwendung**

(30) Priorität: 06.02.1997 DE 19704301
(71) Anmelder: HOECHST AKTIENGESELLSCHAFT, 65929 Frankfurt am Main (DE)
(72) Erfinder: Graulich, Wolff, 35091 Cölbe (DE); Nettelbeck, Dirk, 35037 Marburg (DE); Sedlacek, Hans-Harald, Prof. Dr., 35041 Marburg (DE); Müller, Rolf, Prof. Dr., 35037 Marburg (DE)
(74) Vertreter: Bösl, Raphael, Dr. rer. nat., Dipl.-Ing.

(57) **Zusammenfassung**

Die vorliegende Erfindung bezieht sich auf den Promotor des humanen Endoglingens oder funktionelle Teile davon sowie auf die Verwendung zur Herstellung eines Heilmittels.

SEQUENZLISTE

(1) ALLGEMEINE INFORMATION:

(i) ANMELDER:
(A) NAME: Hoechst Aktiengesellschaft
(B) STRASSE:
(C) ORT: Frankfurt
(D) BUNDESLAND:
(E) LAND: Deutschland
(F) POSTLEITZAHL: 65926
(G) TELEFON: 069-305-3005
(H) TELEFAX: 069-35-7175
(I) TELEX: 412 34 700 ho d

(ii) ANMELDETITEL:
Promotor des humanen Endoglingens sowie seine Verwendung

(iii) ANZAHL DER SEQUENZEN: 3

(iv) COMPUTER LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: Word Perfect 5.1

(2) INFORMATION ZU SEQ ID NO: 1:

(i) SEQUENZ CHARAKTERISTIKA:
(A) LÄNGE: 2415 Basenpaare
(B) ART: Nukleinsõure
(C) STRANDEDNESS: Einzel
(D) TOPOLOGY: linear

(ii) ART DES MOLEKÜLS: DNS (genomisch)

(ix) Merkmale:
(A) NAME/SCHLÜSSEL: exon
(B) LAGE: 1..2415

(xi) SEQUENZBESCHREIBUNG: SEQ ID NO: 1:

CGGGGGTTCC TCCTCTGTAA AGTGGAGGTA TAACGGTACC CACCTCCTGG GGTGGCTGTG
60

AGGATTCAGA GCTGATAAGG TGAACGCCTA GGGCGGGCCC TGGTGCAGAG AGAGCGCTCA
120

GCTCCTAGGG CTGGATTAAC TGTCCCTGGG GCACAGATCT CGGTCTGGGG CCTGTGGAAA
180

CCTCAGAGCC ACCCCTGAAC CCCCACCGAG CCACCCTTTG CCTCGCAGTG CCCATGGCCT
240

CGTCTCCGAG GTTACAGGAA AAGGCAGAGG AGATGCCCTT CTCAGGGTGG CCCTCTGGGA
300

GAGGACACTC TCCCTTGACC TCAAAGCCAC GCTTGGCTGC AAACTGGCCA GGCAGCCACA
360

AGGCTGGGCA AGCAGAACGA TCCCTAATCC CCACCCAAAG AGCCACACCG ACCCTCCCAG

420

CCGCTGTGAC AGCTCCTGCA GAGACAAACA CACGGCCTAC TCTTGTCACC CGGGCCGGCC
480

AATAAGCACG GAGAGGCAAG GCCTCAGACC CTGGACAGAC ATCCTCCCTC CAGAGGCACC
540

AGGGCCTCAG CCTTCTCCTC CCTCCCTGGG CCTCAATTTC TCCACCTGTG ACCCAGGGCA
600

GGTGGATCCA GGGAGAAGAA CCTTCTGGCT CCATCTCACC ATGGGTCCTG CCAGCACACA
660

CAAAGATTTG GCCTCTCAAA GCCTAGCTCT GCCAGCGTCC TTCTGCTCAA GAACTCTCCA
720

TGACTCCCAG TGGCCCTAAG GACAAAGTCC TGGCATTTGA GGCCCTCCCA ATGCAGGGCC
780

AGACTCTGCC TCTCCAGCTT CCTGTCCCCA CCACACCCCT GCTGGTCTCA CGGTGGTCCG
840

ACTGTTTCCT GCTTCTGTGC CTTTGCTTAG TCTGGCACCC CTGCCTGGCA TGCTTTCCTC
900

ACCCCTTCTT CTCCCCAATC CCAACTCACC CAGTCTTTCA AAGGGCAGGC CTAAATACCA
960

GGCCCTCCAG GTGGCCCAGG ATTCCTTCTC TGAGCTTTCA TGGGCCTGGC CCTGGGTGCT
1020

ACCTGTGAGT AGTCCCACGG TGGGTACATA GTAGGTGCGC TTACTGTTCG CAGAATGAAC
1080

ATGGGACAGT TTGGGGACTG TCACCCAGCT CAGGGAGCAC TGATGGGGAA GCATCTCCTG
1140

TATGTCCCAG GGCTCAGTGC TGTAGTGTCC TGACCCTCAG AAATCTCATA ATGGCTTGGT
1200

CAGGAAGGCA TCGTGCCCCA CTTTGCAAAC AGGGGGTGCT GAGAATTGAG GGGCCTTGTC
1260

CAAGGTCTCA TGGCTAGGAG CAAGCAGAAT CGGATTTGAA CCCAGGGCCA CGTGACTTCA
1320

GAAGTGCCAT TAAAGTCCCC ATAATTCGGA GCTGTCTTCT TTTTTTTTTT CTTTCTTTTT
1380

TTTGAGACCG AGCCTCACTC TGTCACCTAG GCCAGGAGTG CAGTGGTCTG ATCTCAGCTC
1440

ACTGCAACCT CCGCCTCCTA GGTTCAAGTG ATTCTCTAGC CTCAGCCTCC CAAGTAGCTG
1500

GGACTACAGG CGCACGTCAT CATGCCCAGC TAACTTTTGT ATTTTTAGTA GAGATGGGTT
1560

TTCACCATGT TGGTCAGGCT GGTCTCGAAC TCCTGACCTC AAGTGATCCG TCTGCCTCGG
1620

CCTCTCAAAG TGCTGGGATT ATAGGCTTGA GCCACTACAC TCGGCCTGGA GCTGTGTTTT
1680

GTCGGTGAAG GATTTTCCAC CCATGAAGGG GTCAGACGTG AAGCGTGTGG CCCTGGGCAG
1740

CTCCTCTGAG CCCAGAGACG CCAGCCCTAG CCGCCTTGCT GTGCCACTTT GGGACTTCCC
1800

TCCCTAGCCT GAGCTTCAGT TTTCCTGCCT GTTAGGCAGC CCCATGTCAA CTGCACTTAG
1860

TAGGCCGGGT TTGATGCCCG ACAAGACGTG AAGTGGTGGA GGTGGGCAGG ATCCCAGCGC
1920

TACCATCTTC TTGAACCAGT GATCTCAACA CATCGGATTT CTGTTTCCTC ATCTGCAAAA
1980

TGGGATCAGT GAGCTCAGGT GGGTCACAAA TTCTACAGGA ACTACTTTAG CCAAGCCCGG
2040

CCCCCTGAAA GTTCCCCTCG GTGGGCAGTT AGGGTGATTG TTTTCATCTG TGGGGCTCCC
2100

TGATGCGTCC CACCCACCAG CCTTGGAGAG GGTGGGATGG GAGGGTGGGG TGCTTGGGGA
2160

GACAAGCCTA GAGCCTGGGC CCTCCCACCC CACTGCCTCC CCCCATCCCA GGGCCCCCCA
2220

CCCAGTGACA AAGCCCGTGG CACTTCCTCT ACCCGGTTGG CAGGCGGCCT GGCCCAGCCC
2280

CTTCTCTAAG GAAGCGCATT TCCTGCCTCC CTGGGCCGGC CGGGCTGGAT GAGCCGGGAG
2340

CTCCCTGCTG CCGGTCATAC CACAGCCTTC ATCTGCGCCC TGGGGCCAGG ACTGCTGCTG
2400

TCACTGCCAT CCATT
2415

(2) INFORMATION ZU SEQ ID NO: 2:

(i) SEQUENZ CHARACTERISTICKA:
(A) LÄNGE: 29 Basenpaare
(B) ART: Nukleinsõure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..29

(xi) SEQUENZBESCHREIBUNG: SEQ ID NO: 2:

GCTGGGCTGG AGTTGCTGTC CGAAGGATG 29

(2) INFORMATION ZU SEQ ID NO: 3:

(i) SEQUENZ CHARAKTERISTIKA:

(A) LÄNGE: 29 Basenpaare
(B) ART: Nukleinsõure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..29

(xi) SEQUENZBESCHREIBUNG: SEQ ID NO: 3:

AATGGATGGC AGTGACAGCA GCAGTCCTG 29

## Beschreibung

Die vorliegende Erfindung bezieht sich auf den Promotor des humanen Endoglingens oder funktionelle Teile davon sowie die Verwendung zur Herstellung eines Heilmittels.

Eine der wesentlichen Probleme der Gentherapie ist die Kontrolle der Transkription und Translation des in die Zelle eingefügten Effektorgenes. Auf der Ebene der Transkription wird diese Kontrolle durch Anfügen einer Promotor- oder Enhancersequenz stromaufwärts der kodierenden Sequenz des Effektorgenes ermöglicht.

Unter der "Promotorsequenz" wird ein Teilabschnitt eines Gens verstanden, an den regulatorische Proteine, die sogenannten Transkriptionsfaktoren, binden können, welche in ihrer Gesamtheit die Transkription des stromabwärts gelegenen Effektorgenes aktivieren. Als "stromabwärts"-Sequenzen werden diejenigen Bereiche bezeichnet, die in Richtung der Transkription liegen, wohingegen Sequenzen, die in der entgegengesetzten Richtung angeordnet sind, als "stromaufwärts"-Sequenzen bezeichnet werden. Unter "Effektorgen" wird im allgemeinen ein Strukturgen verstanden, dessen Genprodukt z.B. einen im Sinne der Gentherapie wünschenswerten Effekt hat.

Derartige Promotor- oder Enhancersequenzen können nicht-zellspezifisch, zellspezifisch, virusspezifisch, metabolisch-spezifisch oder zellzyklusspezifisch sein. In den Patentanmeldungen WO 96/06940, WO 96/06938, WO 96/06941 und WO 96/06939 werden Beispiele für diese Promotorsequenzen und deren Verwendung z.B. für die Gentherapie von unterschiedlichen Erkrankungen aufgeführt. Desweiteren sind in diesen Patentanmeldungen Techniken und Beispiele für die Kombination dieser Promotorsequenzen dargelegt, z.B. für die zellspezifische und zellzyklusspezifische Kontrolle eines Effektorgenes.

Je nach der Auswahl und Kombinationen der Promotoren führen diese zu einer mehr oder weniger eingeschränkten und/oder mehr oder weniger starken Transkription des von diesen Promotoren abhängigen Effektorgenes.

Eine interessante Zielzelle für die Gentherapie ist z.B. die Endothelzelle, zum einen weil Endothelzellen für in das Kreislaufsystem injizierte Genkonstrukte direkt zugänglich sind, zum anderen, weil sie an der Entstehung und dem Verlauf einer Reihe von Krankheiten, wie z.B. Tumorerkrankungen, Entzündungen, Allergien, Autoimmunerkrankungen, Organabstoßungsreaktionen, Kreislauf- und Gerinnungserkrankungen wie auch an Heilvorgängen direkt beteiligt und/oder dem Ort dieser Erkrankungen direkt benachbart sind.

Zielzellspezifische Promotoren sind im Regelfall Promotoren von Genen für solche Proteine, welche in der jeweiligen Zielzelle besonders stark oder weitgehend ausschließlich gebildet werden. Bei der Endothelzelle gehört zu diesen Proteinen beispielsweise Endoglin.

Endoglin ist ein nicht signalübertragender Rezeptor des TGFß (Gougos et al., J. Biol. Chem. 265, 8361 (1990), Cheifetz, J. Biol. Chem. 267, 19027 (1992), Moren et al., BBRC 189, 356 (1992)). In geringen Mengen kommt er auf normalem Endothel vor, ist jedoch verstärkt auf proliferierendem Endothel [Westphal et al., J. Invest. Derm. 100, 27 (1993), Burrows et al., Pharmac. Ther. 65, 155 (1994)] exprimiert. Weiteres ist über die Promotorstärke und Zellspezifität nicht bekannt. Trotz der Tatsache, daß das Endoglingen schon seit ca. 4 Jahren bekannt ist (Bellon et al., (1993), konnte der Endoglinpromotor bis jetzt nicht isoliert werden.

Die cDNA-Sequenz für das menschliche Endoglin wurde von Bellon et al. (Eur. J., Immunol. 23, 2340 (1993)) und für das murine Endoglin von Ge et al. (Gene 138, 201 (1994)) beschrieben. Für einen Teilbereich der 5'-nichttranslatierten Region des Endoglingenes liegen Sequenzinformationen vor, jedoch ist über deren Funktion sowie über den Promotorbereich nichts bekannt.

Ein weiterer Endothelzell-spezifischer Promotor ist der VEGF-Rezeptor. Hierbei werden zwei Rezeptoren unterschieden (Plate et al., Int. J. Cancer 59, 520 (1994)): zum einen der VEGF-Rezeptor-1 (flt-1), (de Vries et al., Science 255, 989 (1992)), der im zytoplasmatischen Teil eine fms-ähnliche Tyrosinkinase enthält und der VEGF-Rezeptor-2 (flk-1, KDR), (Terman et al., BBRC 187, 1579 (1992)), der im zytoplasmatischen Teil eine Tyrosinkinase enthält. Beide Rezeptoren sind fast ausschließlich auf endothelialen Zellen (Senger et al., Cancer Metast. Rev. 12, 303 (1993)) anzutreffen.

Andere Endothelzell-spezifische Rezeptortyrosinkinasen sind tie-1 oder tie-2 (Partanen et al., Mol. Cell. Biol. 12, 1698 (1992), Schnürch und Risau, Development 119, 957 (1993), Dumont et al., Oncogene 7, 1471 (1992)), sowie der B61-Rezeptor (Eck-Rezeptor), (Bartley et al., Nature 368, 558 (1994), Pandey et al., Science 268, 567 (1995), van der Geer et al., Ann. Rev. Cell. Biol. 10, 251 (1994)).

Weitere Endothelzell-spezifische Proteine sind das B61-Molekül, das den Liganden für den B61-Rezeptor darstellt. (Holzman et al., J. Am. Soc. Nephrol. 4, 466 (1993), Bartley et al., Nature 368, 558 (1994)), Endothelin, im speziellen Endothelin B (O'Reilly et al., J. Cardiovasc. Pharm. 22, 18 (1993), Benatti et al., J. Clin Invest 91, 1149 (1993), O'Reilly et al., BBRC 193, 834 (1993)), wobei die Promotorsequenz von Benatti et al., J. Clin. Invest. 91, 1149 (1993) beschrieben wurde, Endothelin-1 (Yanasigawa et al., Nature 332, 411 (1988)), dessen Promotorsequenz von Wilson et al., Mol. Cell. Biol. 10, 4654 (1990) beschrieben wurde, Endothelin-Rezeptoren, insbesondere der Endothelin B-Rezeptor (Webb et al., Mol. Pharmacol. 47, 730 (1995), Haendler et al. J. Cardiovasc. Pharm. 20, 1 (1992)), Mannose-6-Phosphat-Rezeptoren (Perales et al., Eur. J. Biochem. 226, 225 (1994)), deren Promotorsequenzen von Ludwig et al. (Gene 142, 311 (1994), Oshima et al., (J. Biol. Chem. 263, 2553 (1988)) und Pohlmann et al. (PNAS USA 84, 5575 (1987)) beschrieben worden sind, und von Willebrand Faktor (vWF), dessen Promotorsequenz von Jahroudi und Lynch (Mol. Cell. Biol. 14, 999 (1994)), Ferreira et al. (Biochem. J. 293, 641 (1993)) und Aird et al. (PNAS USA 92, 4567 (1995)) beschrieben wurde.

Andere Endothelzell-spezifische Proteine sind IL-1 in Form von z.B. IL-1α und IL-1β, die von aktivierten Endothelzellen produziert werden (Warner et al., J. Immunol. 139, 1911 (1987)), deren Promotorsequenzen von Hangen et al., Mol. Carcinog. 2, 68 (1986), Turner et al., J. Immunol. 143, 3556 (1989), Fenton et al., J. Immunol. 138, 3972 (1987), Bensi et al., Cell Growth Diff. 1, 491 (1990), Hiscott et al., Mol. Cell. Biol. 13, 6231 (1993) und Mori et al., Blood 84, 1688 (1994) beschrieben wurden, IL-1-Rezeptor, dessen Promotorsequenz von Ye et al., PNAS USA 90, 2295 (1993) beschrieben wurde, und Vascular Cell Adhesion Molecule (VCAM-1), wobei die Expression von VCAM-1 in Endothelzellen durch Lipopolysaccharide, TNF-α (Neish et al., Mol. Cell. Biol. 15, 2558 /1995)), IL-4 (Iademarco et al., J. Clin. Invest. 95, 264 (1995)) und IL-5 (Marni et al., J. Clin. Invest. 92, 1866 (1993)) aktiviert wird. Die Promotorsequenz des VCAM-1 wurde von Neish et al., Mol. Cell. Biol. 15, 2558 (1995), Ahmad et al., J. Biol. Chem. 270, 8976 (1995), Neish et al., J. Exp. Med. 176, 1583 (1992), Iademarco et al., J. Biol. Chem. 267, 16323 (1992), und Cybulsky et al., PNAS USA 88, 7859 (1991) beschrieben.

Weitere Endothelzell-spezifische Promotoren sind synthetische Aktivatorsequenzen, wobei als Alternative zu natürlichen endothelspezifischen Promotoren sich auch synthetische Aktivatorsequenzen verwenden lassen, die aus oligomerisierten Bindestellen für Transkriptionsfaktoren, die preferentiell oder selektiv in Endothelzellen aktiv sind, bestehen, beispielsweise der Transkriptionsfaktor GATA-2, dessen Bindestelle im Endothelin-1 Gen 5'-TTATCT-3' ist (Lee et al., Biol. Chem. 266, 16188 (1991), Dorfmann et al., J. Biol. Chem. 267, 1279 (1992) und Wilson et al., Mol. Cell. Biol. 10, 4854 (1990)) und der hirn-spezifische, endotheliale Glucose-1-Transporter, wobei sich Endothelzellen des Hirns durch eine sehr starke Expression dieses Transporter auszeichnen, um den transendothelialen Transport von D-Glucose in das Hirn zu bewerkstelligen (Gerhart et al., J. Neurosci. Res. 22, 464 (1989)). Die Promotorsequenz wurde von Murakami et al. (J. Biol. Chem. 267, 9300 (1992)) beschrieben.

Einige dieser Promotoren sind zwar recht spezifisch für Endothelzellen, aber, wie beispielsweise der Promotor für das Gen des von Willebrand Faktors oder für das Gen des VEGF-Rezeptor-1 (flk-1), nur relativ gering aktiv. Eine Steigerung der Aktivität solcher "schwacher" Promotoren kann zwar durch Kombination mit einem Basalpromotor (z.B. SV40) oder einem Enhancer erreicht werden, jedoch ist dann in der Regel auch eine Verringerung der Spezifität festzustellen.

Aufgabe der vorliegenden Erfindung war es daher, einen Endothel-spezifischen und starken Promoter zu finden.

Überraschenderweise wurde nun gefunden, daß der Endoglingen-Promoter u.a. diese Eigenschaften besitzt.

Ein Gegenstand der Erfindung ist daher der Promotor des menschlichen Endoglingens oder funktionelle Teile und Varianten davon. Es wurde gefunden, daß sich dieser Promotor über maximal 2415 Basenpaare erstreckt (siehe Tabelle 1, SEQ ID NO: 1) und vorzugsweise die Nukleotidsequenz 1-2378 einschließlich der Initiierungssequenz umfaßt.

Zur Charakterisierung des erfindungsgemäßen Promoters wurde die Promotorsequenz des Endoglingenes oder Teile davon im Plasmid pGL3 (Promega) mit einem Reportergen (z.B. Gen kodierend für das Enzym Luziferase) verknüpft und mit diesem Konstrukt Endothelzellen (ECV-304 Zell-Linie) und im Vergleich hierzu Cervixkarzinomzellen (HeLa-Zell-Linie) transfiziert. Überraschenderweise zeigte sich, daß der Endoglinpromotor etwa 80fach stärker als der Promotor des vWF ist. Dies ist deshalb so überraschend, da der vWF - wie oben erwähnt - endothelspezifisch exprimiert wird und somit für den Endoglin-Promotor eine ähnliche Stärke wie für den vWF-Promotor erwartet worden wäre. Ebenfalls zeigte sich, daß der Endoglinpromotor etwa 30fach stärker in Endothelzellen aktiv ist als in Cervixkarzinomzellen. Dies ist deswegen überraschend, da der ebenfalls endothelspezifische vWF-Promotor in Cervixkarzinomzellen eine ähnliche Stärke wie in Endothelzellen hat. Somit wird der vWF-Genpromotor sowohl in bezug auf die Stärke als auch in bezug auf die Endothelspezifität deutlich durch den erfindungsgemäßen Endoglinpromotor übertroffen.

Es zeigte sich des weiteren, daß auch Teile der erfindungsgemäßen Promotersequenz eine starke endothelzellspezifische Aktivität aufweisen.

So ergab sich für die unten aufgeführten 5'-terminal deletierten Konstrukte des Endoglingenpromotors in Endothelzellen folgende relative, auf die Aktivität des SV40 Promoters als Standard bezogene Aktivität:

| Nukleotidsequenz | relative Aktivität |
|---|---|
| SV40 Promotor | 1 |

| Endoglinpromotor (Tab. 1, SEQ ID NO: 1) | |
|---|---|
| 1 -2415 | 11,5 |

| Teilsequenzen: | |
|---|---|
| 36-2415 | 10,2 |
| 470-2415 | 13,0 |
| 948-2415 | 10,0 |
| 1310-2415 | 2,0 |
| 1847-2415 | 3,3 |
| 2339-2415 | 0,2 |

Unter "funktionelle Teile des Promoters" versteht man daher alle Teilsequenzen des erfindungsgemäßen Promoters mit Promoteraktivität, insbesondere die Teilsequenzen von ca. 1 bis ca. 2378, von ca. 36 bis ca. 2378, von ca. 470 bis ca. 2378 und von ca. 948 bis ca. 2378 sowie die Teilsequenzen von ca. 36 bis ca. 2415, von ca. 470 bis ca. 2415 und von ca. 948 bis ca. 2415, vorzugsweise die Teilsequenzen von ca. 470 bis ca. 2415 bzw. von ca. 470 bis ca. 2378. Promotoraktive Teilsequenzen erstrecken sich beispielsweise auch von ca. 1310 bis ca. 2415 bzw. von ca. 1310 bis ca. 2378 und von ca. 1847 bis ca. 2415 bzw. von ca. 1847 bis ca. 2378.

Die vorliegende Erfindung ist jedoch nicht auf den Promotor gemäß SEQ ID NO: 1 und funktionelle Teile davon beschränkt, sondern umfaßt auch Varianten mit Promotoraktivität. Derartige Varianten umfassen beispielsweise Deletionen, Additionen, Insertionen und/oder Substitutionen von ein oder mehreren Basen, vorzugsweise von ca. 1 bis ca. 50, insbesondere ca. 1 bis ca. 25, vor allem ca. 1 bis ca. 5 Basen. Die Promotoraktivität läßt sich leicht, beispielsweise anhand des beschriebenen Luciferase-Assays, messen.

Ein weiterer Gegenstand der vorliegenden Erfindung ist auch ein Nukleinsäurekonstrukt, enthaltend
a) mindestens eine Nukleinsäuresequenz des erfindungsgemäßen Promotors (Komponente a)) und gegebenenfalls
b) mindestens ein Effektorgen (Komponente b)), wobei die Transkription dieses Effektorgenes durch die Komponente a) aktiviert wird.

Die Komponente a) liegt vorzugsweise stromaufwärts von der Komponente b).

Gegenstand der Erfindung ist des weiteren ein Nukleinsäurekonstrukt, in welchem die Promotorsequenz des erfindungsgemäßen Endoglingenes mit einer weiteren zielzellspezifischen, virusspezifischen, metabolisch-spezifischen oder zellzyklusspezifischen Promotorsequenz und mit mindestens einem Effektorgen kombiniert ist und in welchem diese Kombination von Promotorsequenzen die Aktivierung der Transkription mindestens eines Effektorgenes kontrolliert.

Das erfindungsgemäße Nukleinsäurekonstrukt besteht vorzugsweise aus DNS. Unter dem Begriff "Nukleinsäurekonstrukt" werden künstliche Gebilde aus Nukleinsäure verstanden, die sich in den Zielzellen transkribieren lassen. Sie sind bevorzugt in einen Vektor eingefügt, wie beispielsweise in nichtvirale Vektoren wie Plasmide oder virale Vektoren. Die Herstellung von nichtviralen Vektoren wie auch von viralen Vektoren sind dem Fachmann bekannt.

Ein weiterer Gegenstand der vorliegenden Erfindung sind auch Zellen, die ein erfindungsgemäßes Nukleinsäurekonstrukt enthalten.

Die Wahl des Effektorgenes richtet sich im allgemeinen nach der mit dem Genkonstrukt zu behandelnden Erkrankung.

In den Patentanmeldungen WO 96/06940, WO 96/06938, WO 96/06941 und WO 96/06939 werden Beispiele für diese Effektorgene für die Therapie von Tumorerkrankungen, Leukämien, Autoimmunerkrankungen, Allergien, Arthritis, Entzündungen, Organabstoßungen, Transplantat gegen Wirt-Reaktionen, Erkrankungen des Blutgerinnungssystems, Herz-Kreislauferkrankungen, Blutarmut, Infektionen oder Schäden des ZNS ausführlich beschrieben.

Die Effektorgene entsprechend der vorliegenden Erfindung kodieren beispielsweise für ein Cytokin, ein Chemokin, einen Wachstumsfaktor, einen Rezeptor für ein Cytokin, einen Rezeptor für ein Chemokin oder einen Rezeptor für einen Wachstumsfaktor, des weiteren für einen Cytokinantagonisten, ein Zytostase-, Zytotoxizität- oder Apoptose-induzierendes Protein, einen Antikörper oder ein Antikörperfragment, einen Angiogeneseinhibitor, einen Gerinnungsfaktor, einen Gerinnungshemmer, ein fibrinolytisches Protein, ein Enzym, welches eine Vorstufe eines Pharmakons in ein Pharmakon spaltet, ein Protein mit Wirkung auf den Blutkreislauf oder ein Antigen eines Infektionserregers, das eine Immunreaktion bewirkt.

Das erfindungsgemäße Nukleinsäurekonstrukt kann des weiteren zwei oder mehrere gleiche oder unterschiedliche Effektorgene enthalten, welche über Promotorsequenzen oder Internal Ribosomal Entry Sites (IRES) miteinander verknüpft sind. Beispiele hierfür sind in den o.a. Patentanmeldungen dargestellt.

Durch das erfindungsgemäße Nukleinsäurekonstrukt kann ein Gen beispielsweise nur endothelzellspezifisch oder endothelzellspezifisch und metabolischspezifisch, endothelzellspezifisch und zellzyklusspezifisch und/oder endothelzellspezifisch und virusspezifisch exprimiert werden, wobei es sich bei dem Gen bevorzugt um ein Gen handelt, das für einen pharmakologisch aktiven Wirkstoff oder aber für ein Enzym kodiert, welches eine inaktive Vorstufe eines Pharmakons in ein aktives Pharmakon spaltet.

Bevorzugt verwendet wird das erfindungsgemäße Nukleinsäurekonstrukt zur Herstellung eines Heilmittels zur Behandlung der o.g. Erkrankungen, wobei die Herstellung des Arzneimittels im allgemeinen die Klonierung des Nukleinsäurekonstrukts in einen geeigneten Vektor umfaßt, der beispielsweise dem Patienten verabreicht wird. Weitere Anwendungen des erfindungsgemäßen Promotors bzw. des erfindungsgemäßen Nukleinsäurekonstrukts sind dem Fachmann bekannt.

Das folgende Beispiel mit Tabelle und Figuren soll die Erfindung näher beschreiben, ohne sie zu beschränken.

### Beschreibung der Tabelle und Figuren:

- Tabelle 1:: Sequenz des humanen Endoglin-Promotors. Basenpaar 1 entspricht dabei dem am weitesten 5' gelegenen Bereich der Sequenz. Im Bereich der Basenpaare 1360-1666 befindet sich eine hochkonservierte Alu-Sequenz, im 3'-Bereich beginnt bei Basenpaar 2300 die Homologie zur dokumentierten M. musculus cDNA, ab Basenpaar 2379 beginnt der dokumentierte Teil der H. sapiens cDNA (5' nicht translatierter Bereich).

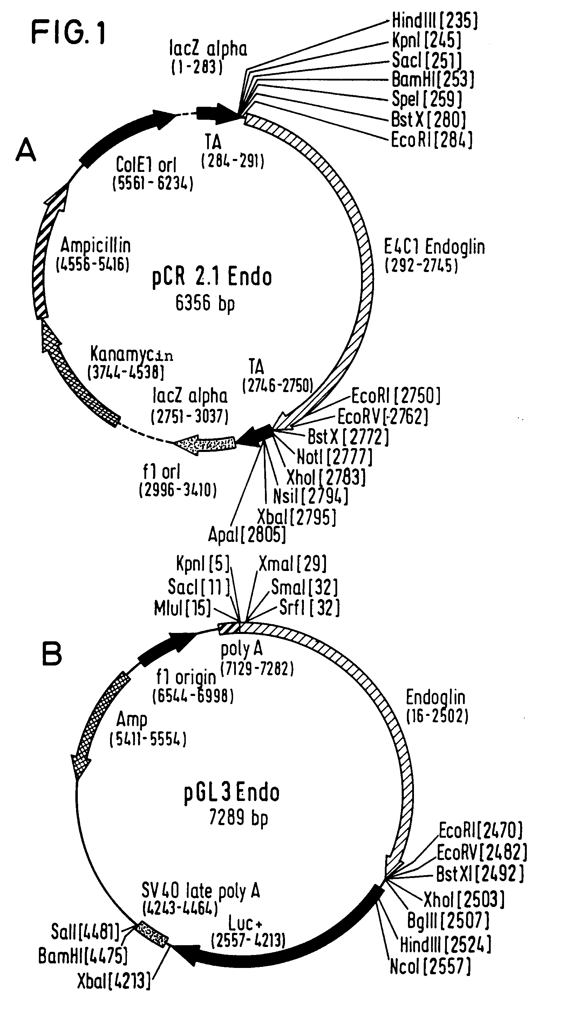

- Fig. 1:: Klonierung des humanen Endoglin-Promotors. A: ein mittels PCR hergestelltes Fragment des humanen Endoglin-Promotors wurde in die TA-Klonierungsstelle des pCR 2.1 Vektors (Invitrogen) ligiert. B: Aus dem Konstrukt pCR 2.1 Endo wurde ein Fragment, welches den humanen Endoglin-Promotor enthält, mit den Enzymen MluI und XhoI herausgeschnitten und in den Luciferase-Reporter Vektor pGL3 (Promega) kloniert.

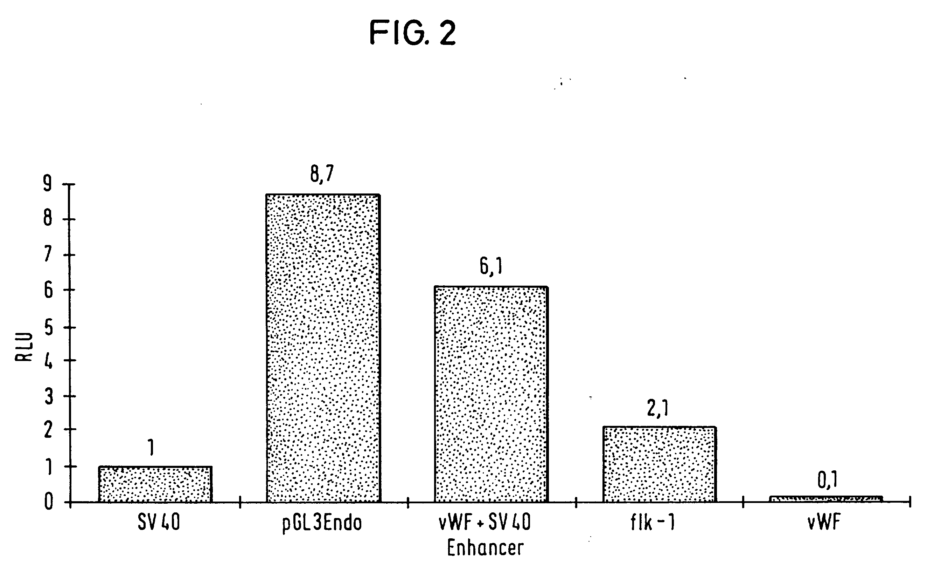

- Fig. 2:: Luciferaseaktivität verschiedener endothelzellspezifischer Promotoren. Alle Promotoren sind in pGL3 kloniert, alle Werte sind auf den SV40 Basalpromotor standardisiert. SV40: Basalpromotor von SV40 ohne Enhancer. pGL3Endo: Endoglin-Promotor (s. Abb. 1B). vWF+SV40 Enhancer: Der Promotor des von Willebrand-Faktors verstärkt mit einem stromaufwärts gelegenen SV40 Enhancer. flk-1: Der Promotor für den VEGF-Rezeptor flk-1 (-224/Start ATG). vWF: Der von Willebrand-Faktor Promotor (-487/+247) ohne zusätzliche Enhancer.

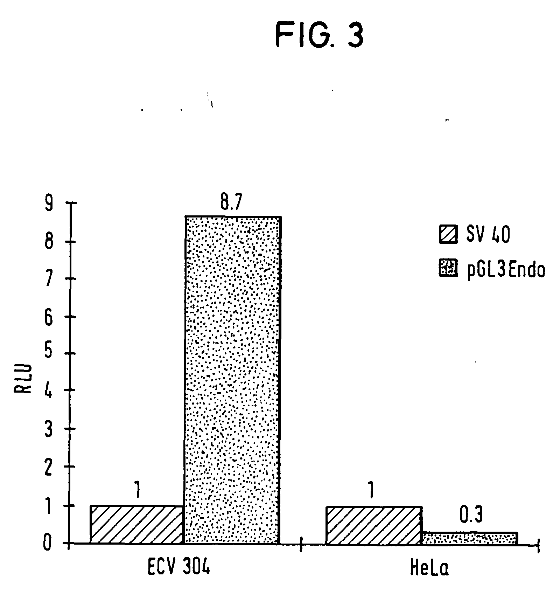

- Fig. 3:: Luciferaseaktivität des humanen Endoglin-Promotors in endothelialen und nicht-endothelialen Zellen. Die Konstrukte sind die gleichen wie in Abb. 2. Auf den Basalpromotor von SV40 standardisiert, wurde die Aktivität des Endoglin-Promotors in der endothelialen Zellinie ECV304 mit der Aktivität in der Cervixkarzinom-Zellinie HeLa verglichen. Die Aktivität in ECV304-Zellen ist in diesem Assay ca. 29 mal höher als in HeLa-Zellen.

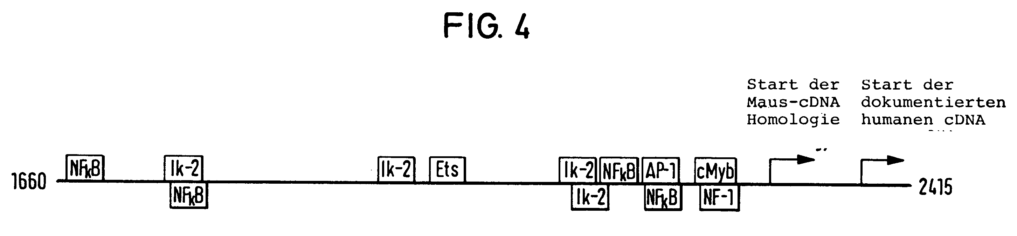

- Fig. 4:: Putative Bindungsstellen für Transkriptionsfaktoren am Endoglin-Promotor. Es ist nur die Region zwischen der Alu-Sequenz und dem Anfang der cDNA gezeigt. Alle Bindungsstellen liegen auf dem Plus(+)-Strang.

### Beispiel

Für die Klonierung des Promotors wurde das PromoterFinder™ DNA Walking Kit (Clontech) verwendet. Mit zwei genspezifischen Primern
E1: GCTGGGCTGGAGTTGCTGTCCGAAGGATG (SEQ ID NO.: 2)
E2: AATGGATGGCAGTGACAGCAGCAGTCCTG (SEQ ID NO.: 3)
aus dem 5' untranslatierten Bereich der humanen Endoglin cDNA wurde damit in zwei PCR-Läufen ein ca. 2,4 Kilobasenpaar-Fragment 5' der dokumentierten Sequenz amplifiziert.

Die PCR-Bedingungen waren dabei:
- 1. PCR:: Primer E1, 25s bei 94°C, 25s bei 94°C, 20s bei 63°C, 4 min bei 68°C, 39 Zyklen 4 min bei 68°C
- 2. (nested) PCR:: Primer E2, 25s bei 94°C, 25s bei 94°C, 20s bei 61°C, 4 min bei 68° C, 26 Zyklen 4 min bei 68°C

### Polymerase: Expand™ Long Template PCR System (Boehringer Mannheim)

Das PCR-Fragment wurde über QIAquick™ Spin Clumns (Qiagen) aufgereinigt und in einen TA-Klonierungsvektor (Original TA Cloning™ Kit (Invitrogen)) eingesetzt. Dieses Konstrukt pCR 2.1Endo (s. Fig. 1a) wurde sequenziert und der klonierte Bereich als 2415 Basenpaar 5'-Region des humanen Endoglingens identifiziert (Sequenz siehe Tabelle 1).

Aus diesem Vektor wurde der klonierte Bereich in einen Luciferase-Reporter Vektor pGL3 (Promega) einkloniert und als Konstrukt pGL3Endo (s. Fig. 1b) in HeLa- sowie ECV304-Zellen auf Promotoraktivität getestet.

Die Zellen wurden nach der DEAE/Dextran-Methode (modifiziert nach Sompayrac et al., PNAS 78, 7575 (1981)) oder mit LipofectAMINE™ (Gibco BRL) transfiziert. Zusätzlich zum Konstrukt pGL3Endo wurde als Standard der SV40 Basalpromotor transfiziert; ferner der flk-1-(VEGF)-(-225/StartATG) Promotor sowie der von Willebrand Faktor-(vWF)-(-487/+247) Promotor ohne oder mit SV40-Enhancer. Dieses vWF-Promotorkonstrukt mit einem SV40-Enhancer hat die Besonderheit, daß seine Aktivität deutlich höher ist als die des Wildtyppromotors, wobei seine Selektivität zwar verringert aber nicht aufgehoben ist. Alle Konstrukte waren in pGL3 kloniert, und der Luciferase-Assay wurde ausgeführt wie in Herber et al. (Oncogene 9, 1295 (1994)) und Lucibello et al. (EMBO J. 14, 132 (1995)) beschrieben.

Die Luciferaseaktivität der verschiedenen Promotoren in ECV304-Zellen (Fig. 2) zeigt, daß das klonierte Fragment der 5'-Region des Endoglingens eine Promotoraktivität besitzt. Diese Aktivität ist sehr hoch, wenn man sie mit den anderen typischen endothelzellspezifischen Promotoren vergleicht. Sie beträgt das vierfache im Vergleich zum flk-1 Promotor und das mehr als achtzigfache im Vergleich zum vWF-Promotor. Selbst bei Verstärkung des vWF-Promotors durch eine SV40-Enhancersequenz ist die Aktivität des pGL3Endo Konstruktes höher. Diese Daten bestätigen, daß es sich bei dem klonierten Bereich um den Promotor des humanen Endoglingens handelt.

In Fig. 3 ist die Aktivität des Endoglin-Promotors in ECV304-Zellen im Vergleich zur Cervixkarzinomzellinie HeLa gezeigt. Standardisiert auf den SV40-Basalpromotor beträgt die Aktivität in ECV304-Zellen rund das 29-fache im Vergleich zu HeLa-Zellen. Dies deutet darauf hin, daß der klonierte Promotor nicht nur in Endothelzellen aktiv, sondern auch für diese selektiv ist.

In Fig. 4 sind putative Bindungsstellen für Transkriptionsfaktoren auf dem Endoglinpromotor dargestellt. In der Region zwischen einer konservierten Alu-Sequenz und der dokumentierten cDNA befinden sich einige stark homologe potentielle Bindungsstellen, welche für die Selektivität und Aktivität des Promotors verantwortlich sein könnten, darunter mehrere konservierte NF- 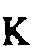 B Bindungsstellen.

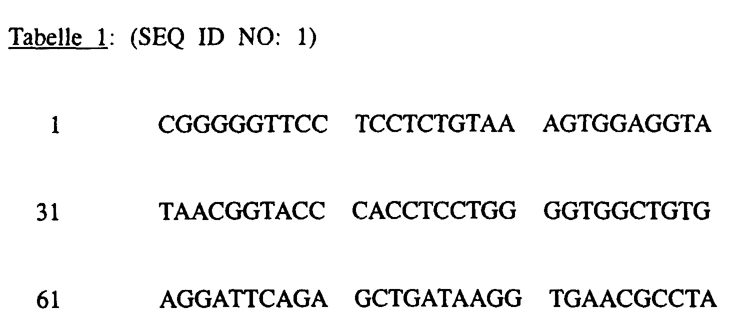

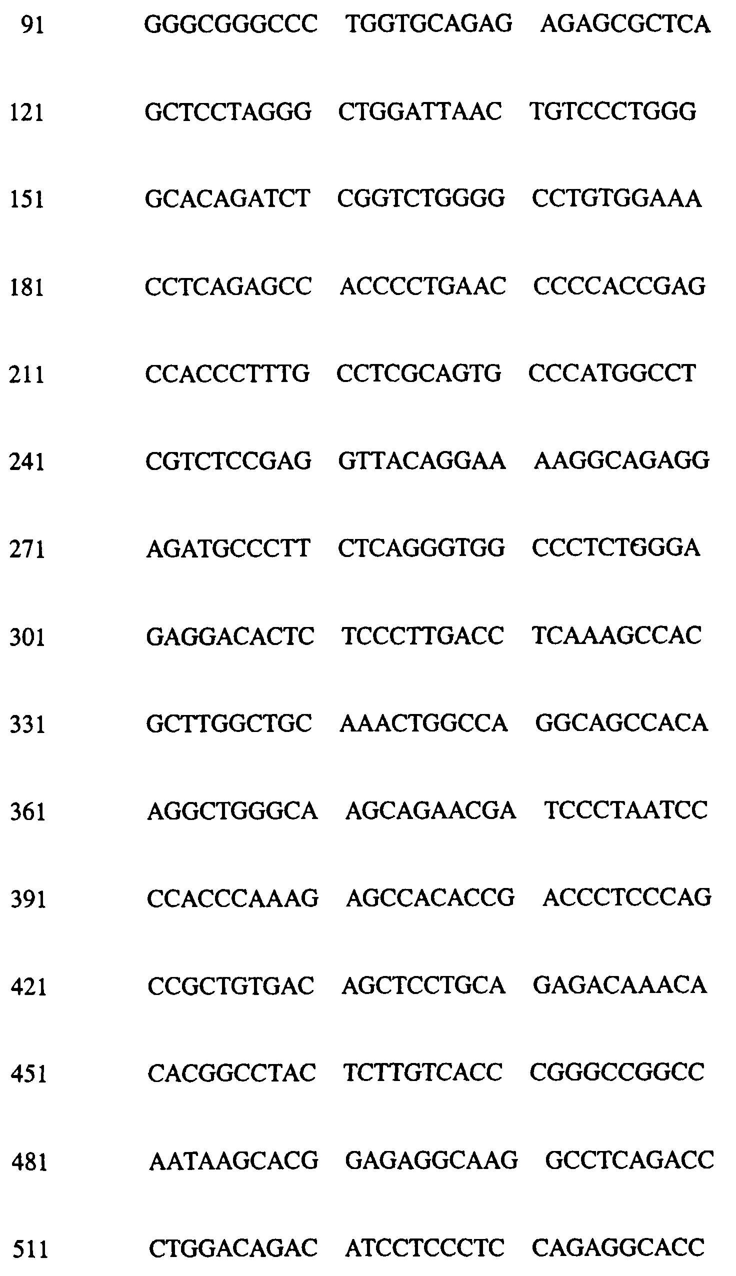

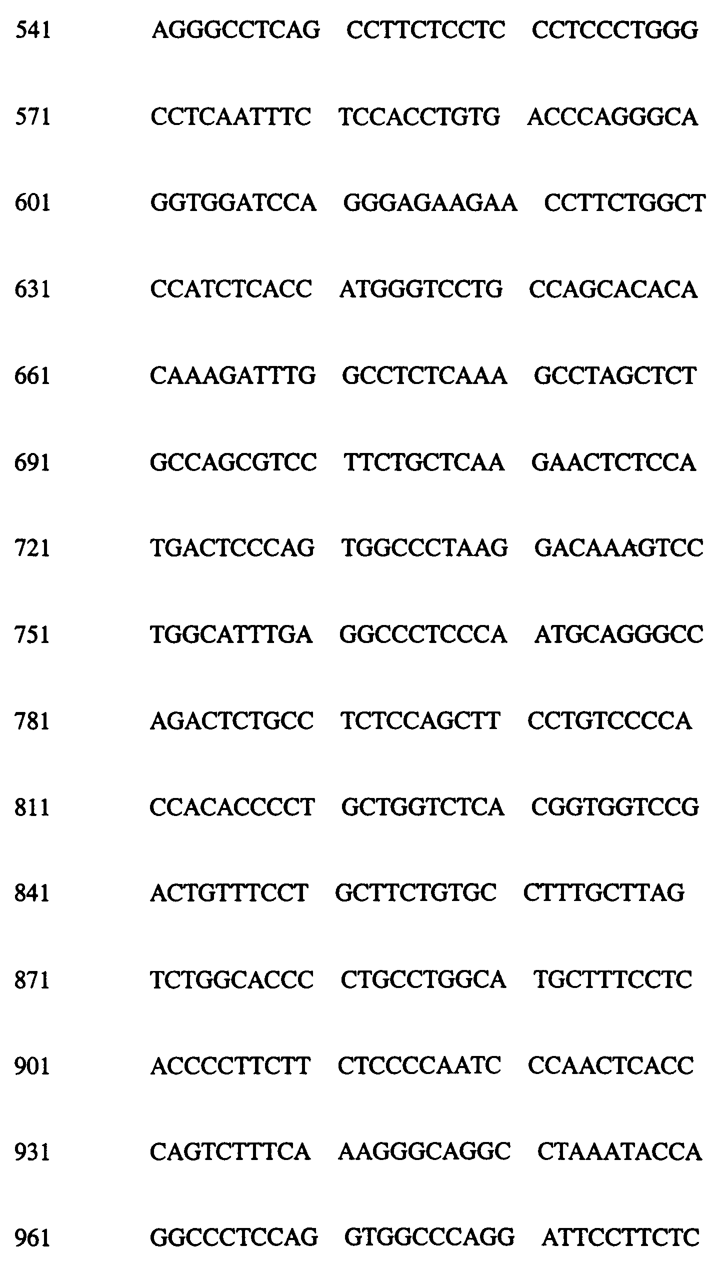

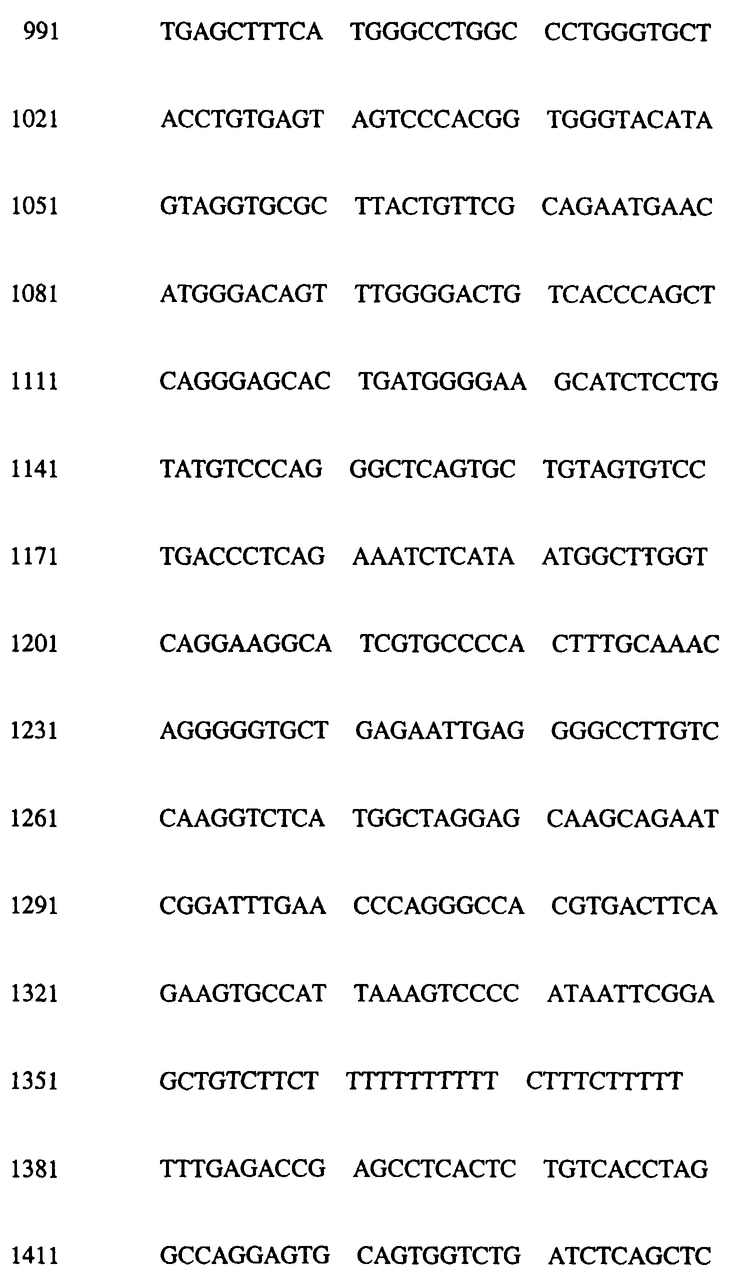

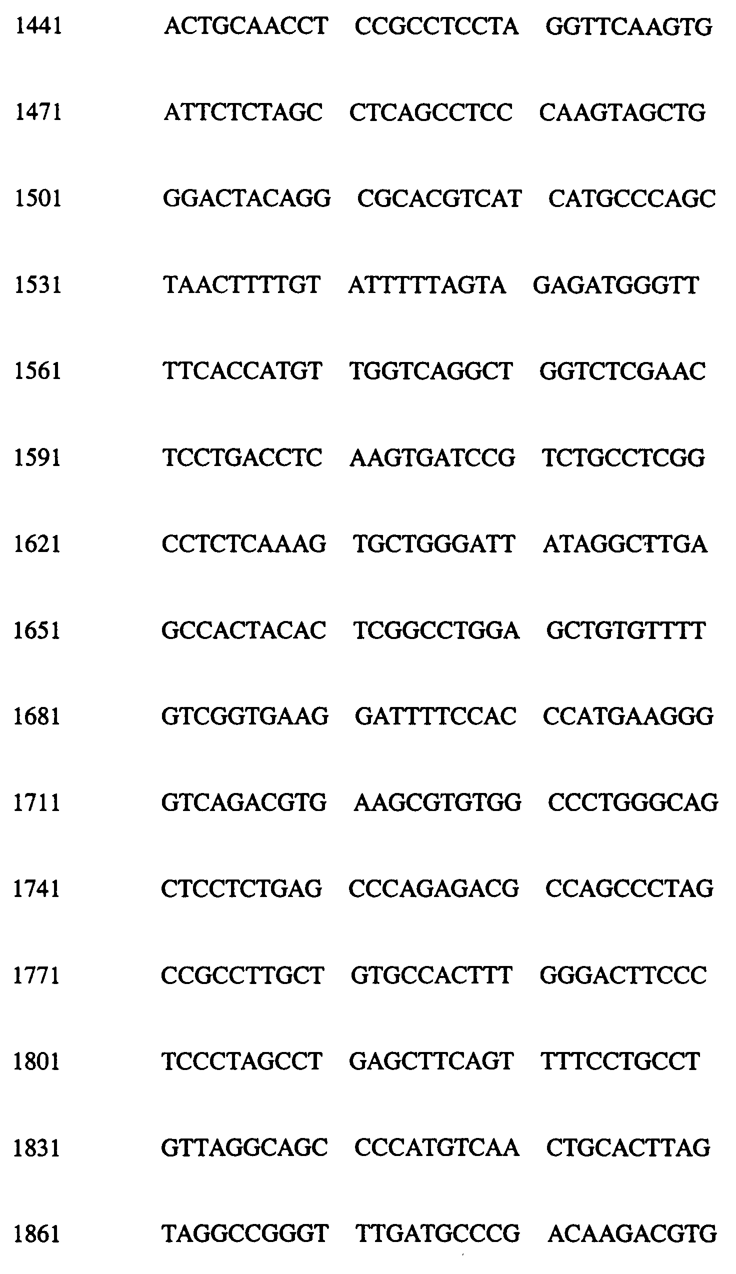

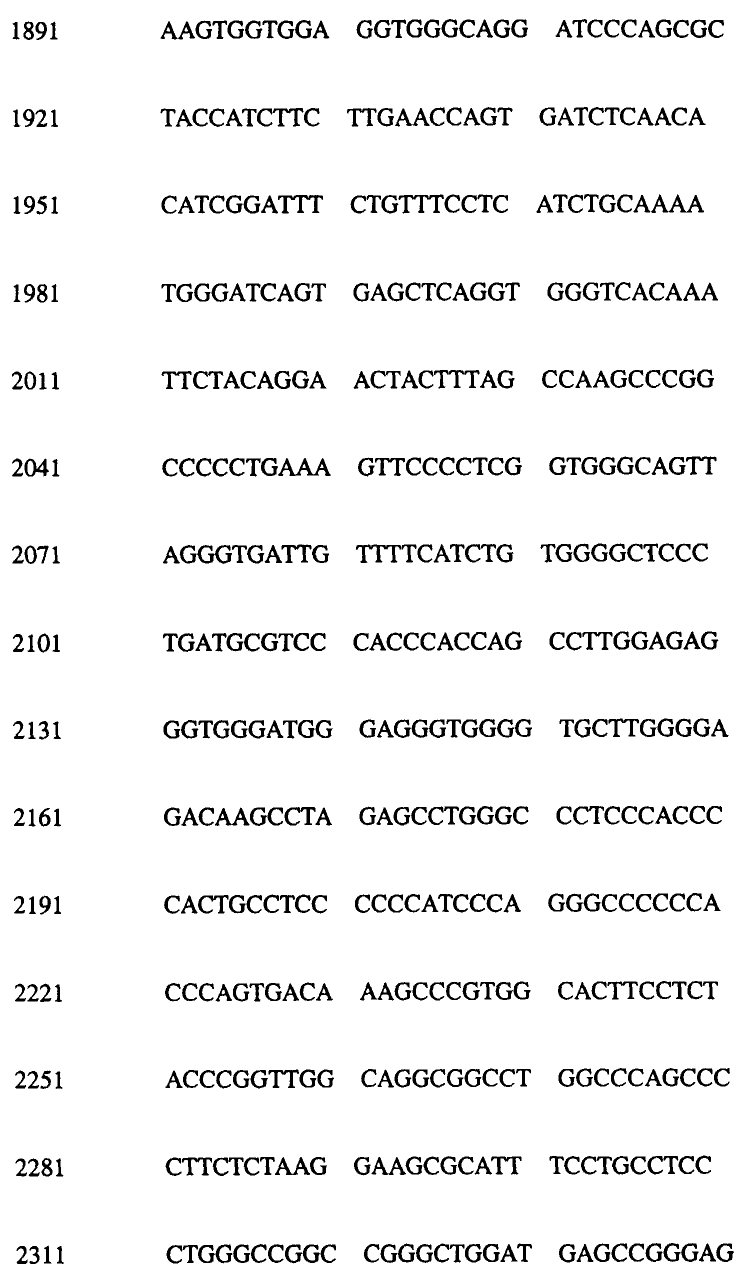

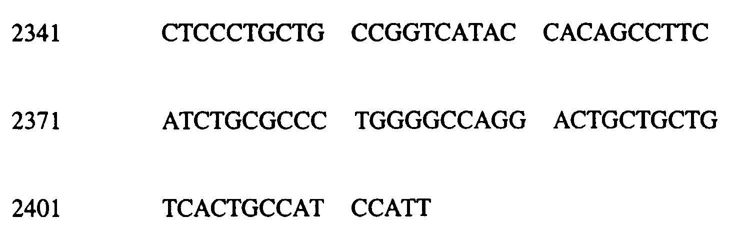

## Patentansprüche

1. Promotor des humanen Endoglingens enthaltend die Sequenz gemäß Tabelle 1 oder funktionelle Teile und Varianten davon.

2. Promotor nach Anspruch 1, dadurch gekennzeichnet, daß die funktionellen Teile die Sequenzen von ca. 1 bis ca. 2378, von ca. 36 bis ca. 2378, von ca. 470 bis ca. 2378, von ca. 948 bis ca. 2378, von ca. 1310 bis ca. 2378, von ca. 1847 bis ca. 2378, von ca. 36 bis ca. 2415, von ca. 470 bis ca. 2415, von ca. 948 bis ca. 2415, von ca. 1310 bis ca. 2415, oder von ca. 1847 bis ca. 2415 umfassen.

3. Nukleinsäurekonstrukt enthaltend mindestens eine Promotorsequenz des humanen Endoglingenes gemäß Anspruch 1 oder 2.

4. Nukleinsäurekonstrukt nach Anspruch 3, dadurch gekennzeichnet, daß es zusätzlich ein Effektorgen enthält, wobei die Promotorsequenz des humanen Endoglingenes (Komponente a) die Transkription des Effektorgenes (Komponente b) aktiviert.

5. Nukleinsäurekonstrukt nach Anspruch 4, dadurch gekennzeichnet, daß die humane Endoglin-Promotersequenz stromaufwärts von dem Effektorgen angeordnet ist.

6. Nukleinsäurekonstrukt nach einem der Ansprüche 3 bis 5, dadurch gekennzeichnet, daß die humane Endoglin-Promotorsequenz mit mindestens einer weiteren Aktivierungsequenz kombiniert ist, wobei diese weitere Aktivierungssequenz ausgewählt ist aus der Gruppe enthaltend eine virusspezifische, metabolisch-spezifische, zellspezifische oder zellzyklusspezifische Aktivierungssequenz.

7. Nukleinsäurekonstrukt nach einem der Ansprüche 3 bis 6, dadurch gekennzeichnet, daß es sich bei der Nukleinsäure um DNS handelt.

8. Nukleinsäurekonstrukt nach einem der Ansprüche 3 bis 7, dadurch gekennzeichnet, daß das Nukleinsäurekonstrukt in einen Vektor eingefügt ist.

9. Nukleinsäurekonstrukt nach Anspruch 8, dadurch gekennzeichnet, daß es sich bei dem Vektor um einen Plasmidvektor oder viralen Vektor handelt.

10. Nukleinsäurekonstrukt nach einem der Ansprüche 4 bis 9, dadurch gekennzeichnet, daß es sich bei dem Effektorgen um bin Gen handelt, das für einen Wirkstoff kodiert, der ausgewählt ist aus der Gruppe umfassend Cytokine, Chemokine, Wachstumsfaktoren, Rezeptoren für Cytokine, Rezeptoren für Chemokine, Rezeptoren für Wachstumsfaktoren, sowie Cytokinantagonisten, antiproliferativ oder zytostatisch oder apoptotisch wirkende Proteine, Antikörper, Antikörperfragmente, Angiogeneseinhibitoren, Gerinnungsfaktoren, Gerinnungshemmer, fibrinolytische Proteine, ein Enzym, welches eine Vorstufe eines Pharmakons in ein Pharmakon spaltet, ein auf den Blutkreislauf wirksames Protein oder ein Antigen eines Infektionserregers, das eine Immunreaktion bewirkt.

11. Nukleinsäurekonstrukt nach einem der Ansprüche 3 bis 10, dadurch gekennzeichnet, daß es mehrere Effektorgene enthält, welche über Promotorsequenzen oder Internal Ribosomal Entry Sites (IRES) miteinander verknüpft sind.

12. Zelle enthaltend ein Nukleinsäurekonstrukt gemäß einem der Ansprüche 3 bis 11.

13. Verwendung eines Nukleinsäurekonstrukts nach einem der Ansprüche 3 bis 10 oder einer Zelle gemäß Anspruch 12 zur Herstellung eines Heilmittels zur Behandlung einer Erkrankung, ausgewählt aus der Gruppe enthaltend Tumorerkrankungen, Leukämien, Autoimmunerkrankungen, Allergien, Arthritiden, Entzündungen, Organabstoßungen, Transplantat gegen Wirt-Reaktionen, Blutgerinnungserkrankungen, Herz-Kreislauferkrankungen, Blutarmut, Infektionen oder ZNS-Schäden.
